# EUROPEAN PATENT APPLICATION

(11) **EP 2 554 663 A2**
(43) Date of publication of application: **06.02.2013**
(21) Application number: 11721797.6
(22) Date of filing: 22.03.2011
(51) Int. Cl.: C12N 5/0797

(54) **METHOD FOR OBTAINING OLIGODENDROCYTE PRECURSOR CELLS**

(30) Priority: 31.03.2010 ES 201030493
(71) Applicant: Fundación del Hospital Nacional de Parapléjicos Para la Investigación y la Integración, 45071 Toledo (ES)
(72) Inventor: DE CASTRO SOUBRIET, Fernando, E-45071 Toledo (ES); ARENZANA SANAGÉRICO, Francisco Javier, E-45071 Toledo (ES)
(74) Representative: Pons Ariño, Angel
(86) International application number: PCT/ES2011/070191
(87) International publication number: WO 2011/121158

(57) **Abstract**

The invention relates to a method for purifying cerebral cortex oligodendrocyte precursor (OP) cells from the cerebral cortex of a mammal that is more than a week old, said method comprising: obtaining an isolated brain sample; selecting the OPs of the previously obtained biological sample; and cultivating the OPs in conditions of cellular proliferation, migration, mobility or differentiation. The invention also relates to the OPs obtained by the method according to the invention and to the cell lines derived using said method.

## Description

The present invention is within the field of neurobiology and biomedicine. It specifically relates to a method for the purification of oligodendrocyte precursor cells (OPCs) of cerebral cortex from samples of mature age.

### STATE OF THE ART

The axons of many vertebrate neurons are isolated by the myelin sheath, which greatly increases the speed at which the axon can conduct the potential of action. Oligodendrocytes are responsible for the formation of myelin in the central nervous system (CNS). These oligodendrocytes envelope the axon layer by layer with their own membrane until forming a narrow spiral, the sheath, which isolates the membrane of the axon so that it barely loses intensity of the electrical impulse. The sheath is interrupted in the Nodes of Ranvier, which are regularly spaced and are points where almost all sodium channels are concentrated. These excellent characteristics of the axon envelope mean that the depolarization of the membrane in a node is extended almost immediately and passively to the following one. Thus, the potential of action is propagated throughout the myelinated axon jumping from node to node. This conduct has two main advantages: the action potentials travel faster and the metabolic energy is conserved as it is only dispersed in small regions of the axon membrane in the Nodes of Ranvier.

The importance of myelination is demonstrated by demyelinating diseases such as multiple sclerosis, wherein the myelin sheath in some regions of the CNS is destroyed by a still unknown mechanism. Oligodendrocytes are post-mitotic cells, totally differentiated, which are not divided *in vivo,* which hinders their long-term *in vitro* culture (Verity et al., 1993 J Neurochem. 60(2):577-87). The OPCs, which have the capacity of proliferating, migrating and differentiating, offer the possibility of studying the molecular and cellular mechanisms of morphofunctional differentiation, as well as the processes of myelination, demyelination and remyelination *in vitro,* and may be a source to promote the myelination and/or the remyelination *in vivo,* with special importance in demyelinating pathologies.

All CNS cells come from neuroepithelial stem cells. These give rise both to neurons and macroglia. The glial cells are astrocytes and oligodendrocytes, which are distinguished by several characteristics. Oligodendrocytes are of smaller size and greater density in their nucleus and their cytoplasm. They lack intermediate filaments and glycogen in their cytoplasm and have a high number of microtubules (Peters et al., 1991, Anat Rec; 229(3):384-98).

The oligodendrocyte may have many cell processes and extensions and each one of them contacts and envelopes a portion of neuronal axon repeated times. When these envelopes are condensed, the membrane spiral forms the myelin sheath. In the same axon, the adjacent segments of myelin belong to different oligodendrocytes and each myelin unit ends up close to a Node of Ranvier (Bunge, 1968, Physiol Rev. 48(1):197-251).

Before maturing until forming the myelin sheath, the oligodendrocytes pass through several stages of development defined by the expression of specific surface receptors and by their response to different growth factors, among other molecules. Thus, the PDGF (platelet-derived growth factor) is associated both to the self-renewal and the generation of oligodendrocytes (Richardson et al., 1988, Nature. 9; 333(6173):562-5.; Raff et al., 1989 Development. 105(3):595-603; Noble et al., 1988 Nature. 9; 333(6173):560-2).

Thus, the OPCs are characterized in that they express Nestin⁺, A2B5⁺, NG2/AN2⁺, plp-dm20⁺, PDGFRα⁺, GD3⁺, CNP⁺. Later, the OPCs give rise to preoligodendrocytes that express the same markers except Nestin and also have 04 (Rowitch, 2004, Nat Rev Neurosci. 5:409-417; de Castro and Bribián, 2005, Brain Res Brain Res Rev. 49(2):227-41)

The start of terminal differentiation (immature oligodendrocyte) is associated to the synthesis and presence on the cell surface of galactosylceramides (GalC), which recognizes the antibody against O1.

The mature oligodendrocytes express myelin basic protein (MBP) and proteolipid protein (PLP) and synthesize the myelin membrane. Other molecular markers expressed by the mature oligodendrocytes are O4⁺, RIP⁺, GalC⁺, ACNP⁺, MAG⁺ (Rowitch, 2004, Nat Rev Neurosci. 5:409-417; de Castro and Bribián, 2005, Brain Res Brain Res Rev. 49(2):227-41).

Although most of the studies of the O-2A progenitors and the mature oligodendrocytes have been carried out in rodents, both O-2A cells and multipolar A2B5⁺/O4⁺ cells, and mature oligodendrocytes O1⁺ have been identified in human foetal brain (Rivkin et al., 1995, Ann Neurol; 38(1):92-101).

In the adult, both oligodendrocytes and OPCs are homogeneously distributed throughout the CNS, mainly in the white matter, but also in the grey matter.

The proliferation and differentiation of the OPCs (including the subsequent myelination process) follow a caudo-rostral gradient throughout the neural tube. Each step of the differentiation process, from the precursor phase to the mature oligodendrocyte, can be identified by changes in the cell morphology and in the expression of immunocytochemical markers, which accompanies a gradual decrease in the proliferative and migratory capacity (Almazán, G et al., 2001, Microsc Res Tech. 15; 52(6):753-65; Rowitch, D, 2004, Nat Rev Neurosci. 5(5):409-19). The expression of these markers even serves to differentiate between various populations of oligodendrocytes. Indeed, the existence of two different populations of OPCs has been proposed: a population dependent on PDGF, a population which we will call PDGFRα⁺, and another independent of this growth factor, population plp/dm20⁺ (Spassky, N et al., 2000, Glia. 15; 29(2):143-8; Spassky, N et al., 1998, J Neurosci. 15;18(20):8331-43.; Pringle, NP and Richarson, WD, 1993, Development.117(2):525-33.; Le Bras, B, 2005, Int J Dev Biol. 49(2-3):209-20). Both oligodendrocyte populations undergo a varied migration pattern to colonize the entire CNS during development.

During development, oligodendrocytes are generated from OPCs. This cell type has been described in the CNS of adult humans and constitutes an interesting source for its use in the treatment of demyelinating lesions, such as, for example, multiple sclerosis. This potential is especially interesting as it has been demonstrated that the axons can be myelinated throughout the life of the neurons, even after having undergone a demyelinating process (Setzu et al.., 2004, Glia 45:307-311). In response to the demyelinating damage, the endogenous OPCs migrate towards the zone of the lesion, but are not capable of penetrating and effectively remyelinating, but they remain in the periphery or, if they penetrate in the lesion zone, they die (Chang et al., 2002, N Engl J Med. 346:165-173).

The study of the OPCs during embryonic development has made it possible to know the molecular mechanisms that regulate migration, proliferation and differentiation of this cell type. However, there are few details about the biology of OPCs in the adult brain, since to date it has only been possible to efficiently isolate OPCs from neonatal embryos. Therefore, it has not been possible to study what is the physiology of these adult OPCs, their similarity and divergences with that seen in embryonic or early postnatal OPCs.

The efficacy of any purification process of a given cell type is inversely proportional to the time elapsed from the obtaining of the sample until the time of the seeding of the cells obtained in the combination of a culture medium and a substrate that allows their survival. The purification of OPCs from embryonic or perinatal tissue is a much more efficient process than from mature or adult tissue, since in the embryonic or young age stage the absolute quantity of the relative proportion of OPCs are significantly greater, and with age, furthermore, the potential for cell survival decreases.

To date, most of the studies have been performed with cultures of OPCs purified from embryonic brain, preferably, or perinatal (from newborn until a maximum of 3-4 days old). There are few studies where OPCs are obtained from adult tissue, with the exception of the rat optical nerve (Shi et al., 1998, J Neurosci. 18(12):4627-4636). This has hindered the study of the neurobiology of adult OPCs, which is vital for the progress of research on demyelinating diseases regarding the search for new pharmacological therapies and the development of regenerative medicine.

Cell lines are a useful tool, frequently used in the search for new drugs. Cell lines are obtained spontaneously or artificially from primary cell cultures. These cells may proliferate indefinitely and are frequently used as models for the study of the cells they come from.

In the case of oligodendroglia, rat oligodendroglial cells have been described derived from 2-day old animal brain cells, such as the CG-4 line, described by Louis et al. in 1992 (J Neurosci Res. 1992; 31(1):193-204) or the OL-1 line, described by Lagarde et al. in 2007 (Int J Dev Neurosci. 2007 25(2):95-105). Human oligodendroglial cell lines come from glioma, oligodendroglioma or oligodendrocytes fused with human tumour cells (Buntinx et al. 2003, J Neurocytol. 32(1):25-38). These cells of tumour origin cannot be used safely in the study of oligodendroglial neurobiology, nor as a therapeutic tool for the treatment of demyelinating diseases, as their immortalization process is unknown. Therefore, a cell line of human OPCs obtained by a controlled immortalization process would be of great use for the performance of drug response tests and cell therapy tests in demyelinating diseases.

The CNS is surrounded by three layers of connective tissue of mesodermal origin known as the meninges (dura mater, arachnoid mater and pia mater), that protect the CNS, among other things, from infections and contain the cerebrospinal fluid (CSF) in the subarachnoid space, also mechanically protecting the CNS. The choroid plexuses are the areas of the cerebral ventricles where the LCR is produced and they are formed by blood capillaries folded over themselves, limited by endothelial cells, coated with relaxed conjunctive tissue and finally coated with an epithelial tissue. For the purification of the OPCs it is necessary to completely remove both the meninges and the choroid plexuses, to avoid the contamination of the culture with cells from other tissues. The purification methods of OPCs described to date resolve the removal of the meninges and the choroid plexuses with an arduous, slow, laborious and delicate dissection with forceps under the stereoscopic microscope, which significantly increases cell death, especially in adult samples.

It is therefore necessary to have a simple procedure that enables the purification of OPCs from mature or adult neural tissue, which allows the study of the neurobiology of this cell population, as well as the performance of drug response tests and cell therapy tests in demyelinating diseases.

### DESCRIPTION OF THE INVENTION

The authors of the present invention provide a method for the isolation and purification of OPCs from mature and adult brains that allows:
i. Studying the neurobiology of adult OPCs, more specifically the processes of proliferation, migration and cell differentiation,
ii. Studying the effects of certain drugs on the OPCs,
iii. Performing molecule screening studies to find new therapeutic targets for demyelinating diseases and
iv. Testing cell therapies on animal models of demyelinating lesions.

Furthermore, the process of the present invention makes it possible to decrease the duration of the cell purification process and considerably increases survival of the OPCs and, with this, the efficacy of the method evaluated in the final number of cells obtained.

This is achieved by eliminating most of the meninges and the choroid plexuses by an enzymatic digestion. As detailed in example 1, the incubation of the previously dissected brains in a solution of the cysteine-protease papain at a concentration (variable according to age) during 5 minutes at 37° C, enables the fast separation of these tissues from the nerve tissue of interest.

Therefore, a first aspect of the invention relates to a method for obtaining OPCs from the cerebral cortex of a mammal of age older than one week, comprising:
a) Obtaining an isolated brain sample,
b) Selecting the OPCs from the biological sample obtained in (a) and
c) Culturing the OPCs in conditions of proliferation or differentiation.

The term "oligodendrocyte precursor cell" or OPC as used in the present invention relates to a cell which has not yet differentiated into a mature oligodendrocyte, and which has potential to differentiate into oligodendrocytes. In one aspect of the invention, the OPC can be characterized in that it has the phenotype Nestin⁺/PSA-NCAM⁺/plp-dm20⁺/PDGFRα⁺. In another aspect of the invention, the OPC can be characterized in that it has the phenotype Nestin⁺/A2B5⁺/NG2/AN2⁺/plp-dm20⁺/PDGFRα⁺/GD3⁺/CNP⁺. Even in another aspect of the invention, the OPC can be characterized in that it has the phenotype A2B5⁺/NG2/AN2⁺/plp-dm20⁺/PDGFRα⁺/O4⁺/GD3⁺/CNP⁺. Nestin, PSA-NCAM, PLP, PDGFRα, A2B5, NG2, 04, GD3 and CNP relate to the expression of cytoplasmic or surface markers that are proteins that are recognized by antibodies against Nestin, PSA-NCAM, PLP, PDGFRα, A2B5, NG2, 04, GD3 and CNP, respectively.

The term "brain" as used in the present invention, relates to mammal neural tissue older than 7 days, including the proencephalon, the hippocampus, the cerebellum, the cortex, the striatum, the telencephalon, the diencephalon, the mesencephalon, the hypothalamus, the locus coeruleus and the rhombencephalon.

The term "mammal" as used in the present invention, is used to refer to any organism in the superkingdom *Eukarya,* kingdom *Metazoa,* phylum *Chordata,* subphylum *Craniata,* superclass *Gnathostomata* and class *Mammalia* to any mammal species, including humans, non-human primates, rodents, equines, canines, felines, bovines, porcines, ovines and lagomorphs.

Preferably, the mammal is more than one week old. More preferably, the mammal is older than two weeks. Even more preferably, the mammal is between 15 days and 9 months old. Much more preferably, the mammal is between 15 days and 6 months old. Preferably, step (b) comprises the enzymatic digestion of the sample obtained according to (a). More preferably, the enzyme used for the digestion of step (b) is papain. Even more preferably, the concentration of papain used for the digestion of step (b) is between 50 and 200 micrograms/millilitre.

In a preferred embodiment, step (b) comprises:
i. A first enzymatic digestion of the sample obtained according to (a),
ii. Removing the meninges and the choroid plexuses,
iii. Mechanically dissociating the nerve tissue resulting from step (ii), and
iv. Enzymatically dissociating the nerve tissue resulting from step (iii).

Preferably, the concentration of papain used in step (i) is between 150 and 200 micrograms/millilitre. More preferably, the concentration of papain used in step (i) is between 160 and 190 micrograms/millilitre. Even more preferably, the concentration of papain used in step (i) is between 170 and 185 micrograms/millilitre.

Preferably, the concentration of papain used in step (iv) is between 50 and 120 micrograms/millilitre. More preferably, the concentration of papain used in step (iv) is between 60 and 110 micrograms/millilitre. Even more preferably, the concentration of papain used in step (iv) is between 70 and 100 micrograms/millilitre.

Preferably, the duration of the enzymatic digestion used in step (b) is between 2 and 22 minutes.

Preferably, the duration of the enzymatic digestion used in step (i) is between 3 and 7 minutes. More preferably, the duration of the enzymatic digestion used in step (i) is between 4 and 6 minutes.

Preferably, the duration of the enzymatic digestion used in step (iv) is between 8 and 18 minutes. More preferably, the duration of the enzymatic digestion used in step (iv) is between 9 and 16 minutes.

Preferably, step (b) comprises an enzymatic digestion of the sample obtained according to (a) with DNase. More preferably, step (b) comprises an enzymatic digestion of the sample obtained according to (a) with DNase-I.

The efficiency of the method of the invention has been assessed by the cell purification method FACS (Fluorescence Activated Cell Sorting) and with the commercial kit called *"Oligodendrocyte Selection Kit"* from Heraclitus Biosciences (HB), as described in examples 1-3. Furthermore, the efficiency of the method of the invention has also been compared to the one described in example 4, where OPCs from the rat optical nerve are purified by immunopanning.

**Table 1. Comparison of the efficiency of the method described by the invention with that of other already described methods (FACS, Kit from Heraclitus Biosciences (Kit HB) and immunopanning).**

| Example | Method | Efficiency in adult (thousands of cells/animal) | Efficiency in perinatal (thousands of cells/animal) |
|---|---|---|---|
| 1 | Invention | 68.5 | 263 |
| 2 | FACS | 10 | - |
| 3 | HB kit | 7.5 | 250 |
| 4 | Immunopanning | 2.5 | - |

As shown in table 1, the efficiency of the method of the invention is much higher than that of any method described to date for the purification of OPCs from mature or adult tissue. The invention presents a method almost 7 times more efficient than the method of the example, almost 10 times more efficient than the method of example 3 and more than 27 times more efficient than the method of example 4.

Another aspect of the invention relates to the cell obtained or obtainable by the method of the invention, hereinafter called "cell of the invention". A preferred embodiment of this aspect of the invention relates to a cell culture derived from the cell of the invention. Another more preferred embodiment relates to a cell line derived from the cell of the invention. More preferably, the cell line has been immortalized by gene transfection.

The term "immortalization" relates to the transformation process of a cell consisting of an acquisition of said cell of dividing itself without limitation in time, without suffering aging or senescence.

The term "gene transfection" relates to any technique or process that allows the integration in a series of cells of a live organism of an exogenous gene, or "transgene", and which gives said cells a new biological property. This process is very well known by a person skilled in the art and may be carried out by viral vectors, by chemical or physical methods. Preferably, the method of transfection is viral infection. The transgene or exogenous gene relates to DNA normally not resident, nor present in the cell to be transformed. In the particular case of the present invention the DNA codes for a cell immortalization gene. Cell immortalization genes can be considered, but without being limited to, the genes that code for: telomerase or the reverse transcriptase of telomerase, myc genes, or viral genes such as SV40T (antigen T of the simian virus 40), EBV (Epstein-Barr virus), adenoviral genes, genes of the Papilomavirus E6 and E7. Another aspect of the invention relates to a method of discovery or screening of drugs comprising:
a. Establishing a cell culture from the OPCs according to claim 16, or a cell line according to claim 17,
b. Administering a chemical substance to the culture or cell line of (a),
c. Studying the effect of the chemical substance of (b) on the cell culture of (a)

The term "studying the effect of the chemical substance" comprises, but without being limited to, studying cell survival, cell viability, cell stress, cell proliferation, cell migration, cell differentiation or studies of demyelination, myelination or remyelination.

Throughout the description and the claims the word "comprises" and its variants are not intended to exclude other technical characteristics, additives, components or steps. For persons skilled in the art, other objects, advantages and characteristics of the invention will be inferred in part from the description and in part from the practice of the invention. The following figures and examples are provided by way of illustration, and are not intended to limit the present invention.

### DESCRIPTION OF THE FIGURES

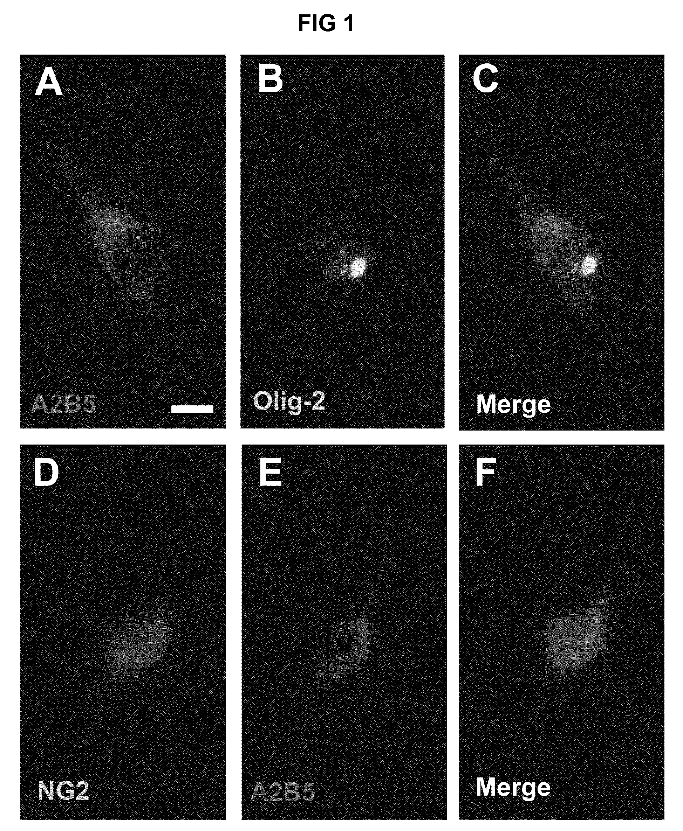

Figure 1. Identification of OPCs of adult mice (60 days old) of the strain CD-1. The OPCs were identified with a double immunocytochemical detection against A2B5 and Olig-2 (**A-C**) or against NG2 and A2B5 (**D-F**). Scale bar: 5 microns.
Figure 2. Separation by FACS of OPCs from adult brain of plp-GFP genetically modified mice. Histograms of the unmarked cells (**A**) and stained with an anti-A2B5 antibody conjugated with phycoerythrin (PE) (**B**). Relative percentages of the different cell populations (GFP⁻/A2B5⁺, GFP⁺/A2B5⁻, GFP⁺/A2B5⁺) unmarked (**C**) and stained with an anti-A2B5 antibody conjugated with PE (**D**).

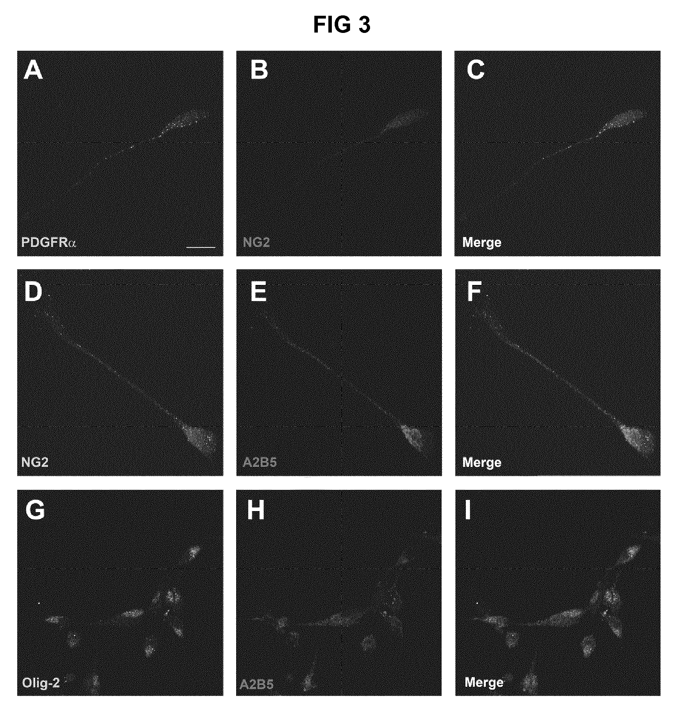

Figure 3. Identification of OPCs from human cerebral cortex from tumour resection margins. The OPCs were identified with a double immunocytochemical stain against PDGFRα and NG2 (**A-C**), against NG2 and A2B5 (**D-F**) and against Olig-2 and A2B5 (**G-I**). Scale bar: 10 microns.

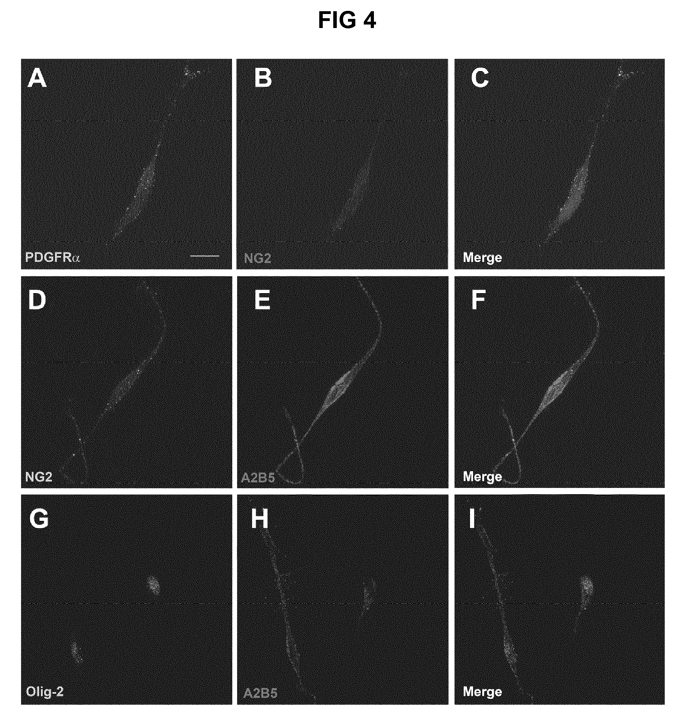

Figure 4. Identification of OPCs from human cerebral cortex obtained from injury resection margins. The OPCs with a double immunocytochemical stain against PDGFRα and NG2 (**A-C**), against NG2 and A2B5 (**D-F**) and against Olig-2 and A2B5 (**G-I**). Scale bar: 10 microns.

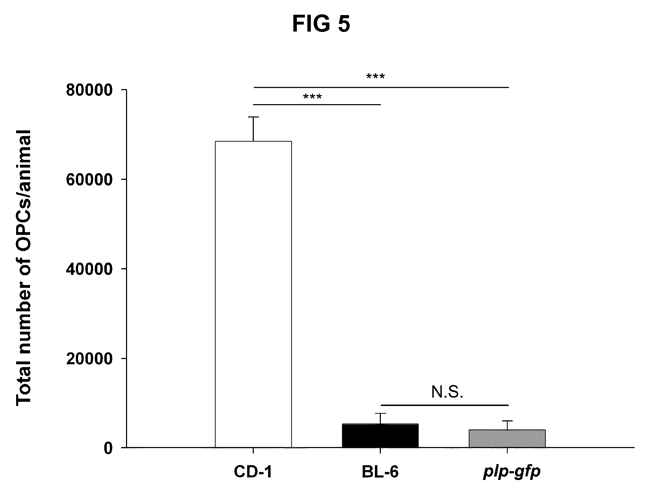

Figure 5. Comparison of the efficiency of our protocol in different mice strains (CD-1, BL-6, plp-GFP). The results were analysed by the *Student t test* using as critical values *P<0.05, **P<0.01 and ***P<0.001.

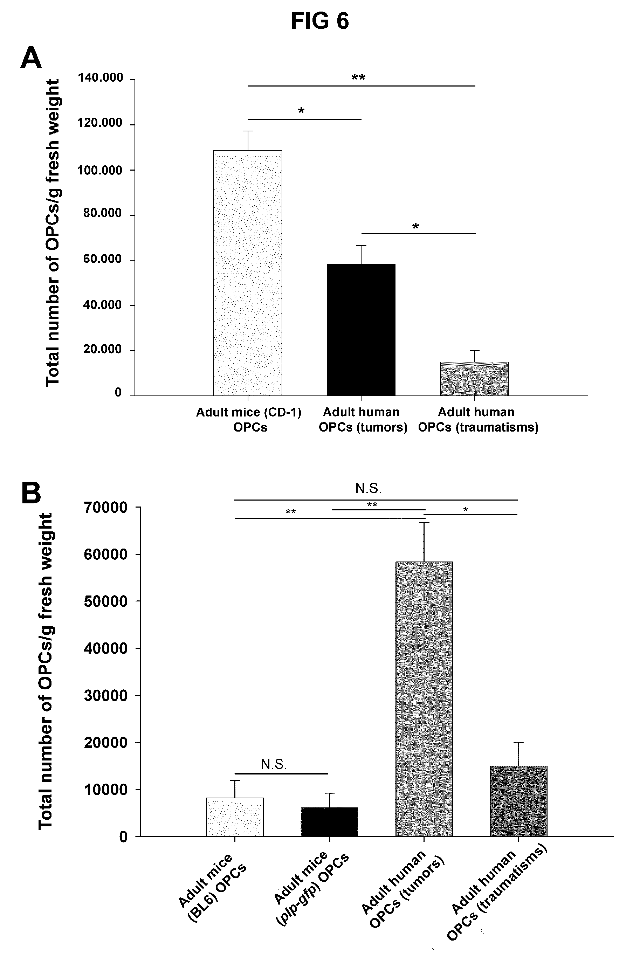

Figure 6. Comparison of the efficiency of the present invention in the mice strain that produced the greatest quantity of OPCs (CD-1) (**A**) and the other strains (**B**) with respect to the two types of biopsies (resection margins of tumours and injuries). In all cases, results were analysed by the *Student t test* using as critical values *P<0,05; **P<0,01 and ***P<0,001.

### EXAMPLES

The following specific examples provided in this document serve to illustrate the nature of the present invention. These examples are only included for illustrative purposes and are not to be interpreted as limitations to the invention claimed herein. Therefore, the examples described below illustrate the invention without limiting the field of application thereof.

### EXAMPLE 1: Purification of OPCs of mouse cerebral cortex of 15 days, 2 months or 6 months old.

The mice of strain CD1 were supplied by Charles River Laboratories. All work was carried out according to the Spanish (RD 1201/2005 (BOE 252/34367-91, 2005)) and European (86/ 609/ EEC and 2003/65/EC) legislations on animal handling and use.

One day before the primary culture, the flasks where the cells were seeded were coated with poly-ornithine at a concentration of 10 mg/l. Before seeding the cells, the poly-ornithine was removed and the flasks were washed to eliminate the remains.

The animals were sacrificed by cervical dislocation and/or asphyxia with CO₂ and the brains were extracted and placed in a dish with saline solution with calcium and magnesium (HBSS or *Hanks' Balanced Salt Solution)* in ice.

To quickly remove the meninges and the choroid plexuses, the brains were incubated in different dilutions of a papain solution (SP, composed of: papain (0.9 mg/ml), L-cysteine (0.2 mg/ml), EDTA (0.2 mg/ml) in HBSS) at 37° C during 5 minutes (see Table 2). The remains of meninges and plexuses were finally removed with forceps and with the aid of a stereoscopic microscope. Once clean, the brain cortexes were taken to a new dish with HBSS in ice.

**Table 2. Summary of the incubation times and the preferred enzymatic concentrations for each postnatal stage analysed and total number of OPCs obtained per animal and relative percentage with respect to the standard age: P0 (newborn).**

| Stage | Animals/flask | 1 st digestion | 2nd digestion | OPCs obtained | relative % of OPCs |
|---|---|---|---|---|---|
| P0 | 3 | SP 1:10 5 min | SP 1:10 5min | 263.148±6.508 | 100 |
| P15 | 5 | SP 1:5 5 min | SP 1:5 10 min | 123.194±8.774 | 50 |
| P60 | 5 | SP 1:5 5 min | SP 1:5 15 min | 68.444±5.461 | 25 |
| P180 | 5 | SP 1:5 5 min | SP 1:5 15 min | 39.757±4.410 | 12 |

The tissue was mechanically dissociated with the aid of scissors and preincubated for 5 minutes at 37° C. The pieces of tissue were incubated in different dilutions of the aforementioned papain solution but in HBSS without calcium or magnesium. The dilution and the time of incubation used for each animal age are detailed in Table 2. The enzymatic digestion was stopped by the addition of medium with serum and DNase-I (DMEM, *Dulbecco's Modified Eagle Medium,* with 10% of foetal bovine serum and 1% of DNase-I).

After mechanically dissociating the tissue via about 25 micropipette runs, the cell suspension was incubated, during 5 minutes, at 37° C and it was then centrifuged at 900 rpm, during 10 minutes. The supernatant and the remains of myelin were eliminated and the cells were resuspended in medium with serum. The cell suspension was filtered through a 100-micron mesh and was seeded in a final volume of 10 ml of OPCs medium (DMEM with 10% foetal bovine serum (SFB), 10 ng/ml PDGFα□ and 1% penicillin and streptomycin) for each flask of 75 cm² of surface, previously coated with poly-ornithine, as described above. The seeding cell density depends on each stage. The number of animals necessary for each flask is detailed in Table 2.

One day later the entire medium was changed to avoid cell death in mature (P15) and adult (P60, P180) samples. In the case of postnatal animals the medium is changed two days later. From then on, the medium was changed every two or three days.

One month or 40 days after seeding a single layer of astrocytes was obtained with OPCs on it. To purify the OPCs, the culture flasks were closed and stirred throughout the night, at 300 rpm and 37° C temperature.

The following morning, the medium was collected with the OPCs in suspension and was filtered through a 40-micron mesh, then centrifuging the cell suspension at 900 rpm during 10 minutes. The cells were resuspended in OPCs medium and seeded in petri dishes during 45 minutes at 37° C to eliminate the microglia cells, which quickly adhere to the substrate. After this time, the cell suspension was collected; it was again filtered through a 40-micron mesh and was centrifuged as before. The cells were resuspended and reseeded in OPCs medium and in petri dishes during another 30 minutes at 37° C to remove the microglia cells that may remain.

This medium enriched in OPCs was collected and centrifuged as described above. The cells were finally resuspended in BS medium (Bottenstein-Sato, for 10 ml of medium: 9.6 ml of DMEM, 100 microlitres of SFB, 100 microlitres of L-Glutamine (200mM), 100 microlitres of Penicillin/Streptomycin, 60 microlitres of 5% BSA, 10 microlitres of transferrin (100mg/ml), 1.86 microlitres of Insulin (50 mg/ml), 1 microlitre of Progesterone (0.62mg/ml), 1 microlitre of putrescine (160 mg/ml), 1 microlitre of T3 3 (mg/ml), 1 microlitre of T4 (4 mg/ml), 1 microlitre of sodium selenite (0.39 ng/ml), counted and seeded in different manners for the study either of proliferation, migration or differentiation to mature oligodendrocytes (Bribián et al., 2006, Mol Cell Neurosci. 33(1):2-14; Merchán et al.., 2007, Mol Cell Neurosci. 36(3):355-68, Bribián et al.., 2008, Dev Neurobiol. 68(13):1503-16).

### EXAMPLE 2: Purification of OPCs of 2-month old mouse cerebral cortex by FACS.

The fluorescence activation cell separation was carried out with *plp*-GFP genetically modified mice (Spassky et al, 1998; Bribián et al, 2006, Merchán et al, 2007).

The animals were sacrificed as in example 1 and the brains were extracted in the same manner. The meninges and the choroid plexuses were removed as in example 1 and the brain cortexes were mechanically dissected with the *"Neural Tissue Dissociation Kit"* from Miltenyi Biotec, according to the manufacturer's instructions.

The remains of myelin were eliminated by filtering the cell suspension through a 40-micron mesh followed by centrifuging in sucrose (0.9 M) in HBSS in ice. The cells were stained with anti-A2B5 antibody conjugated with PE and they were analysed in a FACSAria apparatus from BD. The cells were separated in HBSS with 2% foetal bovine serum, 25 mM HEPES and 5mM EDTA.

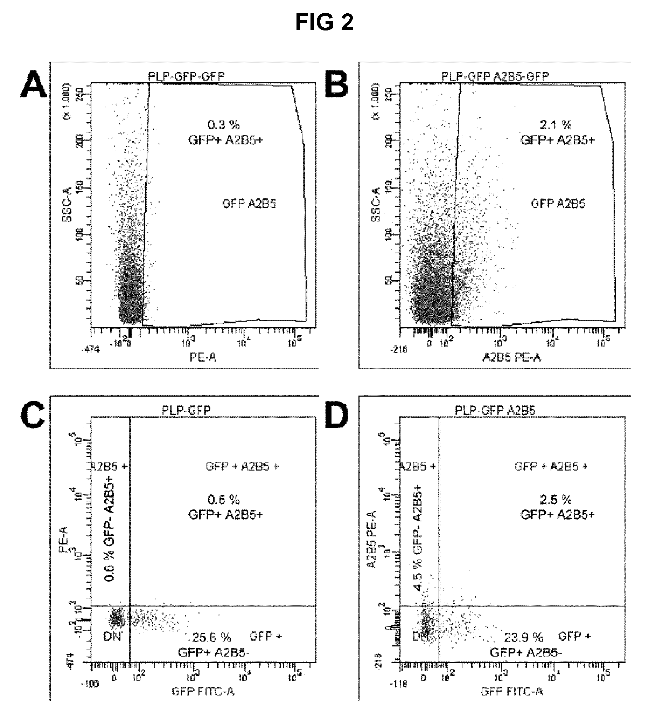

As shown in Figure 2, in the brain of adult mice (2 months old) there is a very low percentage (around 2%) of cells doubly positive for GFP (plp) and A2B5. Furthermore, the separation process causes a mass cell death, since around 50% of the cells analysed survive 24 hours after separation.

The purification of OPCs of 2-month old mouse cerebral cortex by FACS has a final yield of 10,000 OPCs for each animal used.

### EXAMPLE 3: Purification of OPCs of 2-month old mouse cerebral cortex by the "Oligodendrocyte Selection Kit"from HB.

It is important to specify that this kit is described by the manufacturer for the obtainment of immature dendrocytes with O4⁺/GalC⁻ phenotype from P3 rat or mice brains, i.e. 3 days old, which we have previously called perinatal stage. The animals were sacrificed as in example 1 and the brains were extracted in the same manner. Then, the manufacturer's instructions detailed in the user manual were followed.

Despite having introduced the step of enzymatic digestion of the meninges (see above) to minimize cell death, this method (*"Oligodendrocyte Selection Kit"*) was almost 10 times less effective than that described in Example 1, with a final yield of 7,500 OPCs per adult animal.

### EXAMPLE 4: Purification of OPCs of rat optical nerved by immunopanning.

This method is disclosed in patent application PCT/US1996/013279 and consists of the purification of the OPCs of adult rat optical nerve from a mixed suspension of cells from the optical nerve. This cell suspension is first placed in contact with a plate where an anti-Thy1 antibody had previously been absorbed, which enables the removal of non-glial cells. The cell suspension is then placed in contact with a second plate previously absorbed with anti-A2B5n antibody, anti-NG2 antibody or with peanut agglutinin, which is going to permit that only the cells with these proteins on their surface are absorbed in the plate. These cells will mainly be OPCs although, in the case of A2B5, they can also be O-2A precursors.

The efficiency of this method is approximately 2,500 OPCs per each animal (1,250 OPCs per optical nerve; WO/1997/007200; Shi et al., 1998), which is much lower (30 times) than that of the method of the invention (Table 1). EXAMPLE 5: Purification of OPCs of human cerebral cortex from biopsies (resection margins in injuries and tumours).

To obtain human OPCs, the protocol described in example 1 was followed with the following modifications:
a. For the seeding of each 25 cm² flask, a quantity of tissue of between 1 and 4 grams was used, finding the best results using 4 grams.
b. The enzymatic digestion of the meninges and the choroid plexuses was performed using papain solution (SP) at a dilution of 1:2.5, during 10 minutes.
c. The volume of OPCs medium was 5 ml per 25 cm² flask.
d. The stirring to separate the OPCs was performed at 250 rpm.

The efficiency of this method is of 60,000 OPCs for each gram of fresh tissue used in the case of tumour resection margins and 15,000 OPCs for each gram of fresh tissue from injuries.

## Claims

1. A method for obtaining OPCs from the cerebral cortex of a mammal of age older than one week, comprising:
a) Obtaining an isolated brain sample,
b) Selecting the OPCs from the biological sample obtained in (a) and
c) Culturing the OPCs in conditions of proliferation, migration, mobility or cell differentiation.

2. The method according to the preceding claim, where step (b) comprises the enzymatic digestion of the sample obtained according to (a).

3. The method according to claim 2, where step (b) comprises:
i. A first enzymatic digestion of the sample obtained according to (a),
ii. Removing the meninges and the choroid plexuses,
iii. Mechanically dissociating the nerve tissue resulting from step (ii), and
iv. Enzymatically dissociating the nerve tissue resulting from step (iii).

4. The method according to any of claims 2-3, where the enzyme used for the enzymatic digestion of step (b) is papain.

5. The method according to claim 4, where the concentration of papain used in step (b) is between 50 and 200 micrograms/millilitre.

6. The method according to any of claims 4-5, where the concentration of papain used in step (i) is between 150 and 200 micrograms/millilitre.

7. The method according to any of claims 4-6, where the concentration of papain used in step (iv) is between 50 and 120 micrograms/millilitre.

8. The method according to any of claims 4-7, where the concentration of papain used in step (iv) is between 70 and 100 micrograms/millilitre.

9. The method according to any of claims 2-8, where the duration of the enzymatic digestion used in step (b) is between 2 and 22 minutes.

10. The method according to any of claims 3-9, where the duration of the enzymatic digestion used in step (i) is between 3 and 7 minutes.

11. The method according to any of claims 3-9, where the duration of the enzymatic digestion used in step (iv) is between 8 and 18 minutes.

12. The method according to any of claims 1-11, where step (b) comprises an enzymatic digestion with DNase.

13. The method according to any of claims 1-12, where the mammal is older than two weeks.

14. The method according to any of claims 1-13, where the mammal is between 15 days and 9 months old.

15. The method according to any of claims 1-14, where the mammal is between 15 days and 6 months old.

16. OPCs obtainable by the method according to any of claims 1-15.

17. A cell line of OPCs obtainable by the method according to any of claims 1-15, or by cellular immortalization by gene transfection of the OPCs of claim 16.

18. Method of discovery of drugs comprising:
a) Establishing a cell culture from the OPCs, according to claim 16, or a cell line of OPCs, according to claim 17,
b) Administering a chemical substance to the culture or cell line of (a),
c) Studying the effect of the chemical substance of (b) on the cell culture of (a).
